(19) Europäisches Patentamt
European Patent Office
Office européen des brevets

(11) Veröffentlichungsnummer: **0 004 577**
**B1**

(12) **EUROPÄISCHE PATENTSCHRIFT**

(21) Anmeldenummer: **79100758.6**

(51) Int. Cl.³: **C 07 C 47/19** C 07 C 45/00

(22) Anmeldetag: **14.03.79**

(54) **Verfahren zur Herstellung von 2,2-Dimethylolalkanalen**

(30) Priorität: **25.03.78 DE 2813201**

(43) Veröffentlichungstag der Anmeldung:
**17.10.79 Patentblatt 79/21**

(45) Bekanntmachung des Hinweises auf die Patenterteilung:
**01.10.80 Patentblatt 80/20**

(84) Benannte Vertragsstaaten:
**DE FR GB IT SE**

(56) Entgegenhaltungen:
**CH - A - 90 297**
**CH - A - 236 387**
**DE - A - 1 793 512**
**DE - A - 2 507 461**
**DE - A - 2 702 582**

(73) Patentinhaber: **BAYER AG**
**Zentralbereich Patente, Marken und Lizenzen**
**D - 5090 Leverkusen 1, Bayerwerk (DE)**

(72) Erfinder: **Immel, Otto, Dr.**
**Immenhofweg 26**
**D - 4150 Krefeld (DE)**
**Schwarz, Hans-Helmut, Dr.**
**Ratherstrasse 90**
**D - 4150 Krefeld 1 (DE)**
**Quast, Hein, Dr.**
**Campendonkstrasse 1**
**D - 4150 Krefeld (DE)**

**EP 0 004 577 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von 2,2-Dimethylolalkanalen

Die Erfindung betrifft ein Verfahren zur Herstellung von 2,2-Dimethylolalkanalen durch Umsetzung von Formaldehyd mit Aldehyden in Gegenwart von Basen.

Aus der DE—OS 25 07 461 ist bekannt (siehe Vergleichsbeispiel 2), 2,2-Dimethylolbutanal durch Umsetzung von Formaldehyd mit n-Butyraldehyd im Molverhältnis von etwa 3:1 in Gegenwart von Triäthylamin herzustellen. Die Ausbeute an reinem Dimethylolbutanal ist jedoch unbefriedigend.

Weiterhin ist aus der DE—OS 25 07 461 bekannt, 2,2-Dimethylolalkanale durch Umsetzung von Aldehyden mit Formaldehyd in Gegenwart spezieller tertiärer verzweigter Alkylamine herzustellen. Durch die Gegenwart spezieller tertiärer verzweigter Alkylamine bei der Umsetzung wird die Ausbeute an Dimethylolalkanalen gesteigert. Nachteilig ist bei diesem Verfahren jedoch, daß die bei der Kondensation zugesetzten speziellen tertiären verzweigten Alkylamine nur schwer zugänglich sind, wodurch sich das Verfahren weniger wirtschaftlich gestaltet.

Es wurde nun gefunden, daß man in einfacher Weise und mit guter Ausbeute 2,2-Dimethylolalkanale durch Umsetzung von Aldehyden mit Formaldehyd in Gegenwart von Basen erhält, wenn man Aldehyde der Formel

$$RCH_2CHO \qquad (I),$$

in der

R einen gegebenenfalls substituierten aliphatischen Rest bedeutet,

mit Formaldehyd im Molverhältnis von 1:8 bis 1:20 bei Temperaturen im Bereich von 5 bis 100°C in Gegenwart von 0,01 bis 0,5 Mol (pro Mol Aldehyd der Formel (I)) Hydroxiden und/oder Carbonaten der Alkali- und/oder Erdalkalimetalle und/oder unverzweigten tertiären Aminen umsetzt.

Als aliphatische Reste R kommen gegebenenfalls substituierte, geradkettige oder verzweigte Alkylreste mit bis zu 12, insbesondere 1 bis 6, Kohlenstoffatomen in Frage. Als Substituenten dieser Reste kommen unter den Reaktionsbedingungen inerte Gruppen, insbesondere Alkylgruppen oder Alkoxygruppen mit jeweils 1 bis 3 Kohlenstoffatomen in Frage. Beispielsweise seien als Aldehyde der Formel (I) gennant:

3-Äthyl-, 3-n-Propyl-, 3-Isopropyl-, 3-n-Butyl-, 3-Isobutyl-, 3-sek.-Butyl-, 3-tert.-Butyl-butanal sowie entsprechende -n-pentanale, -n-hexanale, -n-heptanale; 4-Äthyl-, 4-n-Propyl-, 4-Isopropyl-, 4-n-Butyl-, 4-Isobutyl-, 4-sek.-Butyl-, 4-tert.-Butyl-pentanale, -n-hexanale, -n-heptanale; 5-Äthyl-, 5-n-Propyl-, 5-Isopropyl-, 5-n-Butyl-, 5-Isobutyl-, 5-sek.-Butyl-, 5-Tert.-Butyl-n-hexanale, -n-heptanale; 3-Methylhexanal, 3-Methyl-heptanal; 4-Methyl-pentanal, 4-Methyl-heptanal, 5-Methyl-hexanal, 5-Methylheptanal; 3,3,5-Trimethyl-n-pentyl-, 3,3-Diäthylpentyl-, 4,4-Diäthylpentyl-, 3,3-Dimethyl-n-butyl-, 3,3-Dimethyl-n-pentyl-, 5,5-Dimethylheptyl-, 3,3-Dimethylheptyl-, 3,3,4-Trimethylpentyl-, 3,4-Dimethylheptyl-, 3,5-Dimethylheptyl-, 4,4-Dimethylheptyl-, 3,3-Diäthylhexyl-, 4,4-Dimethylhexyl-, 4,5-Dimethylhexyl-, 3,4-Dimethylhexyl-, 3,5-Dimethylhexyl-, 3,3-Dimethylhexyl-, 3,4-Diäthylhexyl-, 3-Methyl-4-äthylpentyl-, 3-Methyl-4-äthylhexyl-, 3,3,4-Trimethylpentyl-, 3,4,4-Trimethylpentyl-, 3,3,4-Trimethylhexyl-, 3,4,4-Trimethylhexyl-, 3,3,4,4-Tetramethylpentylaldehyd; bevorzugt sind Propanal, n-Butanal, n-Pentanal, 3-Methylbutanal, n-Hexanal, 3-Methylpentanal, n-Heptanal, 4-Methylhexanal, n-Octanal.

Das erfindungsgemäße Verfahren sei am Beispiel des Butyraldehyds durch nachstehendes Reaktionsschema verdeutlicht.

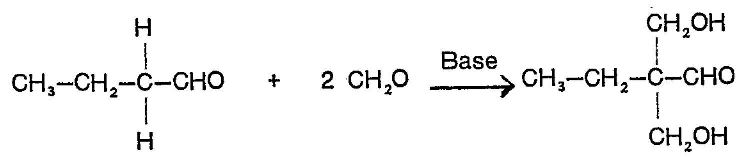


Nach dem erfindungsgemäßen Verfahren kann der Aldehyd der Formel (I) mit Formaldehyd in einem Molverhältnis von etwa 1:8 bis 1:20, besonders bevorzugt im Molverhältnis von 1:10 bis 1:15, in Gegenwart von 0,01 bis 0,5 Mol (pro Mol Aldehyd der Formel (I)) Hydroxiden und/oder Carbonaten der Alkali- und/oder Erdalkalimetalle und/oder unverzweigten tertiären Aminen bei Temperaturen im Bereich von etwa 5 bis 100°C umgesetzt werden.

Formaldehyd wird im allgemeinen als wäßrige Lösung, bevorzugt mit einem Gehalt von 20 bis 40 Gew.-% Formaldehyd, zweckmäßigerweise in handelsüblicher Konzentration, eingesetzt.

Als Hydroxide und Carbonate von Alkali- und Erdalkalimetallen seien z.B. gennant Natriumhydroxid, Kaliumhydroxid, Calciumhydroxid, Bariumhydroxid, Natriumcarbonat und Kaliumcarbonat.

Als tertiäre Amine Kommen unverzwseigte

aliphatische heterocyclische und cycloaliphatische Amine mit bis zu 20 Kohlenstoffatomen, bevorzugt bis zu 15 Kohlenstoffatomen, in Betracht, wobei aliphatische tertiäre Amine bevorzugt eingesetzt werden. Beispielsweise seien folgende tertiäre Amine genannt:

Trimethylamin, Tri-n-propylamin, Triäthylamin, Tri-n-butylamin.

Ebenso unsymmetrische Trialkylamine wie Methyldipropylamin oder Dimethyl-butylamin; Diamine wie N,N-Tetramethyläthylendiamin; N,N-Dimethylcyclohexylamin; Bis-(2-hydroxyäthyl)-cyclohexylamin; N-Methyl-pyrrolidin, N-Methylpiperidin, N-Methyl-morpholin; durch weitere funktionelle Gruppen substituierte Amine wie N,N-Dimethylaminoäthanol, Bis-(2-hydroxy-äthyl)-cyclohexylamin. Weiterhin kommen auch araliphatische Amine wie Tribenzylamin, N,N-Dimethylbenzylamin sowie Polyamine mit tertiären Aminogruppen in Frage wie Triäthylendiamin, Bis-(2-dimethylaminoäthyl)-methylamin; auch Tetraalkylammoniumhydroxide können als Basen eingesetzt werden, z.B. Tetraäthylammoniumhydroxid.

Beim erfindungsgemäßen Verfahren werden die Hydroxide und/oder Carbonate der Alkali- und/oder Erdalkalimetalle und/oder unverzweigte tertiäre Amine bevorzugt in einer Menge von 0,05 bis 0,1 Mol, je Mol Aldehyd der Formel (I) verwendet; der pH-Wert der Reaktionslösung beträgt im allgemeinen 8 bis 13, vorzugsweise 9,5 bis 12,5.

Die angegebenen Mengen an Hydroxiden und/oder Carbonaten der Alkali- und/oder Erdalkalimetalle und/oder unverzweigten tertiären Aminen sind vorteilhaft für das erfindungsgemäße Verfahren. Selbstverständlich können diese Verbindungen auch in Mengen eingesetzt werden, die außerhalb des oben angegebenen Bereiches liegen.

Bei der Durchführung des erfindungsgemäßen Verfahrens kann es vorteilhaft sein, dem Gemisch des Aldehyds der Formel (I) und dem wäßrigen Formaldehyd inerte, organische Lösungsmittel zuzusetzen, um eine bessere Löslichkeit des Aldehyds der Formel (I) in der wäßrigen Formaldehydlösung oder eine homogene Lösung zu erreichen.

Als inerte organische Lösungsmittel kommen die dafür bekannten Lösungsmittel in Frage, bevorzugt niedere aliphatische Alkohole wie Methanol, Äthanol, Propanol und Isopropanol, sowie aliphatische und alicyclische Äther wie Diäthyläther, Tetrahydrofuran und Dioxan.

Die Menge des Lösungsmittels, die zweckmäßigerweise verwendet wird, richtet sich nach der Art des Aldehyds der Formel (I) und kann gegebenenfalls durch einige Vorversuche leicht bestimmt werden.

Die Reaktionstemperaturen beim erfindungsgemäßen Verfahren liegen im allgemeinen im Bereich von etwa 5 bis 100°C, bevorzugt bei 10 bis 60°C, besonders bevorzugt bei 15 bis 35°C.

Das erfindungsgemäße Verfahren kann sowohl diskontinuierlich als auch kontinuierlich durchgeführt werden. Bei der diskontinuierlichen Arbeitsweise kann man z.B. Aldehyde der Formel (I), die Formaldehydlösung und die Hydroxide und/oder Carbonate der Alkali- und/oder Erdalkalimetalle und/oder unverzweigte tertiäre Amine im gewählten Verhältnis und gegebenenfalls das organische Lösungsmittel unter Rühren bei der gewählten Temperatur zusammengeben und das Reaktionsgemisch eine entsprechende Zeit bei der Reaktionstemperatur halten.

Im allgemeinen betragen die Reaktionszeiten zwischen 0,2 und 24 Stunden, insbesondere 1 bis 10 Stunden. Dabei kann die im Einzelfall erforderliche Reaktionszeit in üblicher Weise durch Verfolgen des Reaktionsverlaufs mit analytischen Methoden oder durch einige wenige Vorversuche leicht bestimmt werden.

Im allgemeinen wird das erfindungsgemäße Verfahren bei Normaldruck durchgeführt; es ist jedoch auch möglich, bei vermindertem oder erhöhtem Druck zu arbeiten.

Nach dem erfindungsgemäßen Verfahren können die 2,2-Dimethylolalkanale überraschenderweise bei Verwendung von einfachen und leicht zugänglichen Kondensationsmitteln in guten Ausbeuten erhalten werden. Dadurch gestaltet sich das Verfahren besonders wirtschaftlich als Vorstufe zur industriellen Herstellung von z.B. Trimethylolpropan oder Trimethyloläthan.

Die Trimethylolalkane, z.B. Trimethyloläthan und Trimethylolpropan, die aus 2,2-Dimethylolalkanal durch Reduktion hergestellt werden können, sind Zwischenprodukte von technischer Bedeutung für die Herstellung von Weichmachern, Lackrohstoffen, Polyestern und Polyurethanen. 2,2-Dimethylolalkanale werden auch benötigt für die Herstellung von Dimethylolcarbonsäuren, für Farbstoffe und Schädlingsbekämpfungsmittel (Ullmann's Enzyklopädie der technischen Chemie, Band 3, Seite 295 bis 298).

Das erfindungsgemäße Verfahren sei anhand der folgenden Beispiele erläutert ohne es jedoch auf diese Beispiele zu beschränken.

Beispiel 1

300 kg wäßrige Formaldehydlösung (etwa 30 Gew.-% Formaldehyd, 3000 Mol) und 23,8 kg n-Butanal (330,6 Mol) wurden in einen 500 l Rührkessel gegeben. Unter Rühren wurden im Laufe von 2 Stunden 2,5 kg (25 Mol) Triäthylamin zugesetzt. Die Temperatur wurde auf 20 bis 22°C gehalten. Nach 4 Stunden wurde das Reaktionsgemisch analysiert. Es enthielt 11,8 Gew.-% Dimethylolbutyraldehyd, was einer Ausbeute von 88% entspricht, bezogen auf das eingesetzte n-Butanal.

Beispiel 2

50 kg einer wäßrigen Formaldehydlösung (etwa 30 Gew.-% Formaldehyd) und 3,6 kg (50 Mol) n-Butanal werden in einen Rührkessel

gegeben und mit 250 g (3,4 Mol) $Ca(OH)_2$ versetzt. Der Reaktionskessel wurde gekühlt. Die Temperatur stieg in 28 Minuten von 22 bis 34°C an. Nach einer Reaktionszeit von insgesamt 1 Stunde war die Reaktion beendet. Die Analyse des Reaktionsproduktes ergab umgerechnet eine Ausbeute von 88% der Theorie an Dimethylolbutanal, bezogen auf das eingesetzte n-Butanal.

Beispiel 3

Zu 4000 g (40 Mol) einer 30%igen wäßrigen Formaldehydlösung wurden 60 g (0,81 Mol) $Ca(OH)_2$ gegeben. Das Gemisch wurde gerührt und auf 16°C abgekühlt. Danach wurden 144 g (2 Mol) n-Butanal hinzugesetzt. Die Temperatur stieg dabei auf 21°C an. Nach einer Reaktionszeit von etwa 1 Stunde wurde das Reaktionsprodukt analysiert. Es enthielt 5,7% 2,2-Dimethylolbutyraldehyd, was einer Ausbeute 90,7% entspricht, bezogen auf das eingesetzte n-Butanal.

Beispiel 4

Zu einem Gemisch aus 178 g (3 Mol) Propanal und 3000 g (30 Mol) einer 30%igen wäßrigen Formaldehydlösung wurden unter Rühren innerhalb von 20 Minuten 40 g einer 20 gew.-%igen Natronlauge (0,2 Mol) zugetropft. Dabei stieg die Temperatur von 21 auf 29°C an. 1 Stunde nach Zugabe der Natronlauge wurde das Reaktionsprodukt analysiert. Das Produkt enthielt 10,3% Dimethylolpropanal, was einer Ausbeute von 93% entspricht, bezogen auf das eingesetzte Propanal.

Beispiel 5

72 g (1 Mol) Butyraldehyd, 1500 g (15 Mol) einer 30%igen wäßrigen Formaldehydlösung und 12 g (0,14 Mol) N-Methylpyrrolidin wurden zusammengegeben und 40 Minuten auf Siedetemperatur gehalten. Danach wurden 1256 g der Reaktionsflüssigkeit abdestilliert. Das Destillat wurde mit 8 g (0,094 Mol) N-Methylpyrrolidin versetzt und eine halbe Stunde auf Rückflußtemperatur gehalten. Das so erhaltene Produkt wurde teilweise eingedampft und mit dem Rückstand der ersten Destillation vereinigt. Das Gemisch (714 g) enthielt aufgrund einer Analyse 16,1% 2,2-Dimethylolbutanal, was einer Ausbeute von 87% entspricht, bezogen auf das eingesetzte n-Butanal.

Beispiel 6

72 g (1 Mol) n-Butanal, 1500 g (15 Mol) einer 30%igen wäßrigen Formaldehydlösung und 20 g (0,115 Mol) Bis-(2-dimethylaminoäthyl)-methylamin wurden 20 Minuten unter Benutzung eines Rückflußkühlers auf Siedetemperatur gehalten. Das Reaktionsprodukt enthielt aufgrund einer Analyse 6,9% Dimethylolbutanal, was einer Ausbeute von 83% entspricht, bezogen auf das eingesetzte n-Butanal.

Beispiel 7

72 g (1 Mol) n-Butanal, 1000 g (10 Mol) einer 30%igen wäßrigen Formaldehydlösung und 50 g (0,05 Mol) einer 13,8%igen wäßrigen $K_2CO_3$-Lösung wurden vereinigt und gerührt, wobei die Temperatur auf 41°C anstieg und allmählich wieder auf Raumtemperatur abfiel. Nach 24 Stunden wurde das Reaktionsprodukt analysiert: Es enthielt 9,9% Dimethylolbutanal, was einer Ausbeute von 84% entspricht, bezogen auf das eingesetzte n-Butanal.

Beispiel 8

216 g (3 Mol) n-Butanal, 3000 g (30 Mol) einer 30%igen wäßrigen Formaldehydlösung und 30 g (0,3 Mol) Triäthylamin wurden zusammengegeben und 20 Minuten auf Siedetemperatur gehalten. Danach wurde die Reaktionsflüssigkeit andestilliert, wobei 2169 g Destillat ebgetrennt wurden. Das Destillat wurde mit 10 g (0,1 Mol) Triäthylamin versetzt und eine halbe Stunde auf Rückflußtemperatur gehalten. Dieses Produkt wurde anschließend mit einer 30 cm Füllkörperkolonne bei Normaldruck destilliert. Der Destillationsrückstand (475 g) wurde mit dem Rückstand der ersten Destillation vereinigt. Das Gemisch (1545 g) enthielt aufgrund einer Analyse 22,8% Dimethylolbutanal, was einer Ausbeute von 89% entspricht, bezogen auf das eingesetzte n-Butanal.

Beispiel 9

4000 g (40 Mol) einer wäßrigen Formaldehydlösung (etwa 30 Gew.% Formaldehyd) 29,3 g (0,4 Mol) $Ca(OH)_2$ und 60,3 ml (0,4 Mol) einer 40%igen wäßrigen Trimethylaminlösung wurden zusammengegeben und 15 Minuten gerührt. Sodann wurden 292 (4 Mol) n-Butanal hinzugesetzt. Dabei stieg die Temperatur kurzzeitig von 25° auf 36°C an und fiel dann wieder auf 26°C ab. Nach einer Reaktionszeit von 2 Stunden enthielt das Reaktionsgemisch aufgrund einer Analyse 10,8% 2,2-Dimethylolbutanal, was einer Ausbeute von 89,6% entspricht, bezogen auf das eingesetzte n-Butanal.

Beispiel 10

Zu 4000 g (40 Mol) einer 30%igen wäßrigen Formaldehydlösung wurden 60 g (0,81 Mol) $Ca(OH)_2$ gegeben und 15 Minuten gerührt, bevor 432 g (6 Mol) n-Butanal hinzugesetzt wurden. Durch Kühlung des Reaktionsgefäßes wurde die Temperatur zwischen 10 und 15°C gehalten. Nach einer Reaktionszeit von 3 Stunden enthielt das Reaktionsgemisch aufgrund einer Analyse 14,8% 2,2-Dimethylolbutyraldehyd, was einer Ausbeute von 84% entspricht, bezogen auf das eingesetzte n-Butanal.

Beispiel 11

Zu einem Gemisch aus 22,4 kg (31,1 Mol) n-Butanal und 387,6 kg (387,6 Mol) einer 30%igen wäßrigen Formaldehydlösung wurden 1,05 kg (14 Mol) $Ca(OH)_2$ eingerührt. Der Reaktionskessel wurde gekühlt und so auf 20—24°C gehalten. Nach einer Reaktionszeit von 5 Stunden enthielt das Reaktionsgemisch aufgrund einer Analyse 8,7% 2,2-Dimethylolbutanal, was einer Ausbeute von 87% entspricht, bezogen auf das eingesetzte n-Butanal.

Beispiel 12

Zu 4000 g (40 Mol) einer 30%igen wäßrigen Formaldehydlösung wurden 12 g $Ca(OH)_2$ und 72 g einer 20%igen Natronlauge gegeben. Nach einer Rührzeit von 15 Minuten wurden 288 g (4 Mol) n-Butanal hinzugesetzt. Dabei stieg die Temperatur im Reaktionsgefäß von 21 auf 35°C an. Durch äußere Kühlung wurde die Reaktionstemperatur auf 32 bis 35°C gehalten. Nach einer Reaktionszeit von 1 Stunde enthielt das Reaktionsgemisch aufgrund einer Analyse 10,8% 2,2-Dimethylolbutanal, was einer Ausbeute von 88% entspricht, bezogen auf das eingesetzte n-Butanal.

## Patentansprüche

1. Verfahren zur Herstellung von 2,2-Dimethylolalkanalen durch Umsetzung von Aldehyden mit Formaldehyd in Gegenwart von Basen, dadurch gekennzeichnet, daß man Aldehyde der Formel

$$RCH_2CHO \quad (I),$$

in der

R einen gegebenenfalls substituierten aliphatischen Rest bedeutet,

mit Formaldehyd im Molverhältnis von 1:8 bis 1:20 bei Temperaturen im Bereich von 5 bis 100°C in Gegenwart von 0,01 bis 0,5 Mol (pro Mol Aldehyd der Formel (I)) Hydroxiden und/oder Carbonaten der Alkali- und/oder Erdalkalimetalle und/oder unverzweigten tertiären Aminen umsetzt.

2. Verfahren nach Anspruch 1, dadurch gekennzeichnet, daß man als Verbindungen der Formel (I) Propionaldehyd und Butyraldehyd einsetzt.

3. Verfahren nach Ansprüchen 1 bis 2, dadurch gekennzeichnet, daß man die Umsetzung des Aldehyds der Formel (I) mit Formaldehyd bei einem pH-Wert der Reaktionslösung im Bereich von 8 bis 13 durchführt.

## Claims

1. Process for the preparation of 2,2-dimethylolalkanals by reacting aldehydes with formaldehyde in the presence of bases, characterized in that aldehydes of the formula

$$RCH_2CHO \quad (I)$$

in which

R denotes an optionally substituted aliphatic radical,

are reacted with formaldehyde in a molar ratio from 1:8 to 1:20 at temperatures in the range from 5 to 100°C and in the presence of hydroxides and/or carbonates of alkali metals and/or alkaline earth metals, and/or tertiary amines.

2. Process according to Claim 1, characterised in that propionaldehyde and butyraldehyde are employed as compounds of the formula (I).

3. Process according to Claims 1 to 2, characterised in that the reaction of the aldehyde of the formula (I) with formaldehyde is carried out at a pH value of the reaction solution in the range from 8 to 13.

## Revendications

1. Procédé de production de 2,2-diméthylolalcanals par réaction d'aldéhydes avec le formaldéhyde en présence de bases, caractérisé en ce qu'on fait réagir des aldéhydes de formule:

$$RCH_2CHO \quad (I)$$

(dans laquelle

R est un reste aliphatique éventuellement substitué)

avec le formaldéhyde dans un rapport molaire de 1:8 à 1:20 à des températures comprises dans la plage de 5 à 100°C en présence de 0,01 à 0,5 mole (par mole d'aldéhyde de formule I)) des hydroxydes et/ou des carbonates des métaux alcalins et/ou alcalino-terreux et/ou d'amines tertiaires non ramifiées.

2. Procédé suivant la revendication 1, caractérisé en ce qu'on utilise comme composés de formule (I) le propionaldéhyde et le butyraldéhyde.

3. Procédé suivant l'une des revendications 1 et 2, caractérisé en ce qu'on conduit la réaction de l'aldéhyde de formule (I) avec le formaldéhyde à un pH de la solution réactionnelle compris dans la plage de 8 à 13.